# EUROPEAN PATENT APPLICATION

(11) **EP 2 253 321 A1**
(43) Date of publication of application: **24.11.2010**
(21) Application number: 09006166.4
(22) Date of filing: 06.05.2009
(51) Int. Cl.: A61K 31/525, A61K 31/14, A61K 45/06, A61P 27/02, A61P 41/00

(54) **Collyrium for the treatment of conial cornea with "cross-linking" trans-epithelial technique**

(71) Applicant: Pinelli, Roberto, 25124 Brescia (IT)
(72) Inventor: Pinelli, Roberto, 25124 Brescia (IT)

(57) **Abstract**

Collyrium for the treatment for patients suffering from conical cornea containing the riboflavin photosensitising substance and a surface-active agent to assist penetration of the collyrium in the corneal epithelium; compared with normal collyria for the treatment of conical cornea, the product according to the invention has the advantage of not requiring the removal of the corneal epithelium, with the absence of pain for the patient, who does not need particular post treatment therapy, no longer complains of edema due to the removal of the epithelium and preserves the original corneal transparency.

## Description

### BACKGROUND OF THE INVENTION

The present invention relates to an improved collyrium for the treatment with "cross-linking" trans-epithelial technique of patients suffering from conical cornea.

In people suffering from conical cornea, the cornea is weaker and the normal links between the collagen fibers are much less numerous.

Conical cornea is a progressive pathology and bilateral in almost all cases, i.e. it affects both the eyes of the patient.

Within the aim of stiffening the corneal structure and assisting an increase of the links between the corneal collagen fibers (cross-linking) it is practice to instill riboflavin in collyrium form onto the cornea and photosensitize it with a source of UVA rays.

Collyria presently known for this purpose require, for the treatment to be effective, that the corneal epithelium is removed, with consequent periods of corneal edema (opacity of the cornea) post-treatment and lengthy re-epithelisation of the part treated.

### SUMMARY OF THE INVENTION

The aim of the present invention is to provide a new collyrium for the treatment of these patients that, unlike those existing, does not require removal of the corneal epithelium.

This aim is achieved with a collyrium containing the riboflavin photosensitizing substance and a surface-acting agent that assists penetration of the collyrium into the corneal epithelium. Preferred embodiments result from the sub claims.

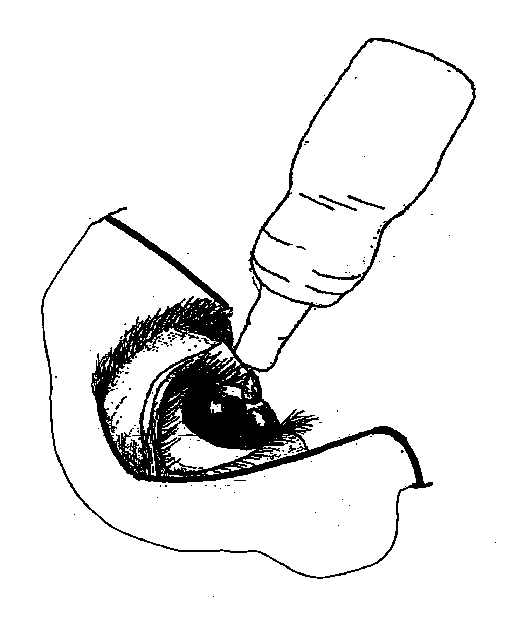

Other aims, advantages and characteristics result from the collyrium realized according to the invention, here described with reference to several examples of preparation of the same. The administering procedure of the collyrium, devised according to the present invention, results from the enclosed drawing.

The collyrium of the present invention contains riboflavin, i.e. vitamin B₂ present in nature in food such as fruit and vegetables.

Riboflavin has photosensitizing capacities whereby, in the presence of ultraviolet rays, it accumulates energy and transfers it to surrounding means.

Therefore, when it is instilled onto the cornea (corneal stroma), which is composed essentially of water and collagen fibers, and then affected by a source of UVA rays (3 mW/cm² source and rays of a wavelength equal to 370 nm), the riboflavin first accumulates energy and then transfers it to the water molecules causing the hydrolisis thereof; the ions originating from this process (free radicals) link to the collagen fibers and reinforce the structure (cross-linking).

The traditional need to remove the corneal epithelium is overcome by the presence in the collyrium subject matter of the invention of a surface-active compound that has the task of making the riboflavin penetrate through the epithelium.

The preferred surface-active agent for the invention is in particular Benzal Chloride.

Hereinafter a formulation example of the collyrium is indicated prepared according to the present invention; the quantities are specified for 100 ml of solution:
150 mg riboflavin-5-sodium phosphate (powder)
440 mg Sodium Chloride (powder)
900 mg Sterile water (liquid)
20 mg Benzal Chloride

## Claims

1. Collyrium for the treatment of conical cornea with "cross-linking", trans-epithelial technique, **characterized in that** it contains riboflavin provided with photosensitizing properties and a surface-active compound that assists the penetration of the riboflavin into the corneal epithelium.

2. Collyrium, according to claim 1, **characterized in that** the surface-active agent is composed of Benzal Chloride.

3. Use of collyrium, according to claims 1 and 2, **characterized in that** it is applicable in the treatment of patients suffering from conical cornea.

4. Use of collyrium, according to claim 3, **characterized in that** the photo-sensitization of the riboflavin occurs with the assistance of UVA rays.

5. Use of collyrium, according to claim 4, **characterized in that** the photo-sensitization is realized with a source of UVA rays of 3 mW/cm² source and rays of a wavelength equal to 370 nm.

6. Collyrium for the treatment of conical cornea, according to claim 1, **characterized by** the following composition for 100 ml of solution:
150 mg riboflavin-5-sodium phosphate (powder)
440 mg Sodium Chloride (powder)
900 mg Sterile water (liquid)
20 mg Benzal Chlorid
